# EUROPÄISCHE PATENTANMELDUNG

(11) **EP 1 445 003 A1**
(43) Veröffentlichungstag der Anmeldung: **11.08.2004**
(21) Anmeldenummer: 04001092.8
(22) Anmeldetag: 20.01.2004
(51) Int. Cl.: A61N 7/00, A61B 17/22

(54) **Stosswellengenerator mit vergrössertem Fokus**

(30) Priorität: 04.02.2003 DE 10304434
(71) Anmelder: Dornier MedTech Systems GmbH, 82234 Wessling (DE)
(72) Erfinder: Bohris, Christian, Dr., 82152 Krailling (DE)
(74) Vertreter: Grünecker, Kinkeldey, Stockmair & Schwanhäusser Anwaltssozietät

(57) **Zusammenfassung**

Die Erfindung betrifft einen Stoßwellengenerator mit einer Linse, wobei die Linse bezüglich der akustischen Achse eine nichtrotationssymmetrische Phasenverschiebungscharakteristik aufweist. Die Erfindung betrifft außerdem die Verwendung einer Linse mit einer nichtrotationssymmetrischen Phasenverschiebungscharakteristik für Stoßwellen.

## Beschreibung

Die Erfindung betrifft einen Stoßwellengenerator, bei dem die Fokuszone aufgeweitet ist.

Stoßwellengeneratoren bzw. Schockwellengeneratoren werden beispielsweise in Therapieeinrichtungen zur Behandlung von Steinleiden (Lithotripsie), Tumorleiden und Knochenleiden (Osteorestauration) verwendet. Zur Zertrümmerung beispielsweise von Nierensteinen werden Stoßwellensequenzen von einer Stoßwellenquelle erzeugt, die auf das Konkrement im Körper fokussiert werden.

Aus der US 5 800 365 ist eine elektrodynamische Stoßwellenquelle bekannt, die eine primäre und eine sekundäre Stoßwelle mit einer spezifischen Zeitverzögerung erzeugt, um auf diese Weise die Zertrümmerungswirkung durch Kavitation zu vergrößern.

Aus der US 5 873 845 ist ein System zur Ultraschall-Erwärmung von biologischem Gewebe bekannt, das eine Refraktionsplatte zur Aufweitung der Fokusregion umfasst. Hintergrund der Erfindung ist die Verwendung kontinuierlicher Ultraschallwellen ("continuous wave") zur Koagulierung von biologischem Gewebe; es handelt sich nicht um Stoßwellen.

Ein wichtiges Anliegen bei der Gestaltung der Stoßquellen besteht darin, auf der einen Seite die Effektivität der Zertrümmerung zu verbessern und auf der anderen Seite die Nebenwirkungen durch die nicht vom Stein absorbierte akustische Energie zu reduzieren. Herkömmliche Stoßwellenquellen erzeugen üblicherweise geometrisch und zeitlich exakte Pulse, die zu einer sehr scharfen Bündelung führen. Eine scharfe Bündelung hat häufig die Konsequenz, dass das Konkrement in mehrere, immer noch verhältnismäßig große Stücke gebrochen wird. Die resultierende Zertrümmerung ist wenig effektiv. Außerdem ergeben sich auf Grund der Kavitationsneigung unerwünschte Nebenwirkungen.

Es ist daher die Aufgabe der Erfindung, einen Stoßwellengenerator bereitzustellen, der eine hohe Zertrümmerungseffektivität bei geringen Nebenwirkungen aufweist.

Diese Aufgabe wird gelöst durch einen Stoßwellengenerator gemäß Anspruch 1.

Der erfindungsgemäße Stoßwellengenerator umfasst eine Linse, wobei die Linse bezüglich der akustischen Achse eine nichtrotationssymmetrische Phasenverschiebungscharakteristik aufweist.

Die nichtrotationssymmetrische Phasenverschiebungscharakteristik bzw. zeitliche Verzögerungscharakteristik ermöglicht eine zeitliche Verzögerung von Teilen der Stoßwellenfront, wodurch die Spitzendrücke in der Fokusachse gesenkt werden können. Insbesondere kann auf diese Weise der Fokus aufgeweitet und die Pulslänge verlängert werden. Damit erreicht man sowohl eine Verbesserung der Zertrümmerungswirkung als auch eine Verringerung der Nebenwirkungen. Eine mögliche Hypothese zur Erklärung der verbesserten Zertrümmerungswirkung beruht darauf, dass bei einem breiten Fokus der sog. Squeezing-Effekt (W. Eisenmenger, "The mechanics of stone fragmentation in ESWL", Ultrasound Med. Biol. 2001; 27: 683 - 693) verstärkt auftritt, der sehr effizient bei der Steinzertrümmerung wirkt.

Die nichtrotationssymmetrische Phasenverschiebungscharakteristik kann kontinuierlich ausgebildet sein. Um die Herstellung einer entsprechenden Linse zu vereinfachen, kann die nichtrotationssymmetrische Phasenverschiebungscharakteristik gemäß einer vorteilhaften Alternative nichtkontinuierlich ausgebildet sein.

Um eine besonders vorteilhafte Phasenverschiebung zu erzielen, kann die Linse wenigstens zwei Sektoren mit gleicher Phasenverschiebungscharakteristik aufweisen. Die Linse wird somit in wenigstens zwei Kreissektoren aufgeteilt, wobei die Linse in jedem Kreissektor mit gleicher Phasenverschiebungscharakteristik ausgebildet ist. Dies bedeutet, dass sich die gleiche Phasenverschiebungscharakteristik in jedem Kreissektor wiederholt.

Gemäß einer vorteilhaften Weiterbildung aller zuvor beschriebenen Stoßwellengeneratoren kann die Linse eine nichtrotationssymmetrische Dickenverteilung zum Ausbilden der nichtrotationssymmetrischen Phasenverschiebungscharakteristik aufweisen. Damit kann in einfacher Weise eine Linse mit einer gewünschten Phasenverschiebungscharakteristik hergestellt werden.

Vorzugsweise kann die Linse eine nichtkontinuierliche Dickenverteilung aufweisen. Eine Dickenverteilung, bei der sich die Dicke in diskreten Schritten ändert, erlaubt insbesondere eine einfache Herstellungsweise der Linse.

Gemäß einer vorteilhaften Weiterbildung kann die Linse eine Mehrzahl von radial ausgerichteten, keilförmigen Elementen zum Ausbilden der nichtrotationssymmetrischen Dickenverteilung aufweisen. Die keilförmigen Elemente müssen nicht notwendigerweise im Zentrum der Linse beginnen, sie können auch erst ab einem bestimmten Abstand von dem Zentrum bzw. der optischen Achse ausgebildet sein.

Gemäß einer vorteilhaften Weiterbildung aller zuvor beschriebenen Stoßwellengeneratoren kann die Dicke als Funktion des Winkels über einen vorherbestimmten Winkelbereich linear ansteigen. Der Winkelbereich kann beispielsweise einen Sektor umfassen, so dass die Dicke in dem Sektor als Funktion des Winkels linear ansteigt. Der lineare Anstieg muss nicht notwendigerweise kontinuierlich sein, sondem kann auch in diskreten Schritten erfolgen, indem die Linse beispielsweise keilförmige Elemente umfasst, die in der Art von Treppenstufen linear ansteigende Höhen bzw. Dicken aufweisen.

Gemäß einer vorteilhaften Weiterbildung aller zuvor beschriebenen Stoßwellengeneratoren kann die Linse wenigstens zwei Materialien umfassen. Durch eine geeignete Wahl der Materialien mit bestimmten Schallgeschwindigkeiten können auf diese Weise Linsen mit gewünschten Phasenverschiebungscharakteristika hergestellt werden. Diese unterschiedlichen Materialien können statt oder als Ergänzung einer nichtkontinuierlichen Dickenverteilung der Linse eingesetzt werden. Beispielsweise kann eine Linse Abschnitte aus unterschiedlichen Materialien umfassen.

Vorzugsweise kann die Linse einen formstabilen Werkstoff, insbesondere Polistyrol, umfassen. Dieses Material weist verschiedene Vorteile auf. Zum Einen ergeben sich wegen der akustischen Impedanz und der kleinen Dämpfungskonstante geringe Verluste auf Grund von Reflexion und Dämpfung in der Linse. Außerdem erlaubt der Unterschied in der Schallgeschwindigkeit zwischen Polistyrol und Wasser signifikante Phasenverschiebungen mit geringen Veränderungen der Linsendicke, wodurch die Dämpfung in der Linse weiter reduziert wird. Im Übrigen ist Polistyrol ein billiges und leicht zu verarbeitendes Material.

Die Erfindung stellt außerdem die Verwendung einer Linse mit einer nichtrotationssymmetrischen Phasenverschiebungscharakteristik für Stoßwellen bereit. Die Verwendung einer solchen Linse für Stoßwellen, insbesondere bei der Fokussierung von Stoßwellen, erlaubt eine vorteilhafte Aufweitung des Fokus, wie bereits oben beschrieben wurde.

Die bevorzugten Weiterbildungen entsprechen den zuvor beschriebenen Weiterbildungen des erfindungsgemäßen Stoßwellengenerators.

Die nichtrotationssymmetrische Phasenverschiebungscharakteristik kann kontinuierlich ausgebildet sein. Um die Herstellung einer entsprechenden Linse zu vereinfachen, kann die nichtrotationssymmetrische Phasenverschiebungscharakteristik gemäß einer vorteilhaften Alternative nichtkontinuierlich ausgebildet sein.

Um eine besonders vorteilhafte Phasenverschiebung zu erzielen, kann die Linse wenigstens zwei Sektoren mit gleicher Phasenverschiebungscharakteristik aufweisen.

Gemäß einer vorteilhaften Weiterbildung kann die Linse eine nichtrotationssymmetrische Dickenverteilung zum Ausbilden der nichtrotationssymmetrischen Phasenverschiebungscharakteristik aufweisen.

Vorzugsweise kann die Linse eine nichtkontinuierliche Dickenverteilung aufweisen.

Gemäß einer vorteilhaften Weiterbildung kann die Linse eine Mehrzahl von radial ausgerichteten, keilförmigen Elementen zum Ausbilden der nichtrotationssymmetrischen Dickenverteilung aufweisen. Die keilförmigen Elemente müssen nicht notwendigerweise im Zentrum der Linse beginnen, sie können auch erst ab einem bestimmten Abstand von dem Zentrum bzw. der optischen Achse ausgebildet sein.

Gemäß einer vorteilhaften Weiterbildung aller zuvor beschriebenen Linsen kann die Dicke als Funktion des Winkels über einen vorherbestimmten Winkelbereich linear ansteigen. Der Winkelbereich kann beispielsweise einen Sektor umfassen, so dass die Dicke in dem Sektor als Funktion des Winkels linear ansteigt. Der lineare Anstieg muss nicht notwendigerweise kontinuierlich sein, sondern kann auch in diskreten Schritten erfolgen, indem die Linse beispielsweise keilförmige Elemente umfasst, die in der Art von Treppenstufen linear ansteigende Höhen bzw. Dicken aufweisen.

Gemäß einer vorteilhaften Weiterbildung aller zuvor beschriebenen Linsen kann die Linse wenigstens zwei Materialien umfassen. Durch eine geeignete Wahl der Materialien mit bestimmten Schallgeschwindigkeiten können auf diese Weise Linsen mit gewünschten Phasenverschiebungscharakteristika hergestellt werden.

Vorzugsweise kann die Linse einen formstabilen Werkstoff, insbesondere Polistyrol, umfassen.

Weitere Merkmale und Vorteile der Erfindung werden im Folgenden an Hand der Zeichnung beschrieben.
- Figur 1: zeigt eine schematische Darstellung eines Ausführungsbeispiels einer Linse für einen erfindungsgemäßen Stoßwellengenerator und
- Figur 2: zeigt eine schematische Darstellung der Fokusanordnung für das Ausführungsbeispiel aus Figur 1.

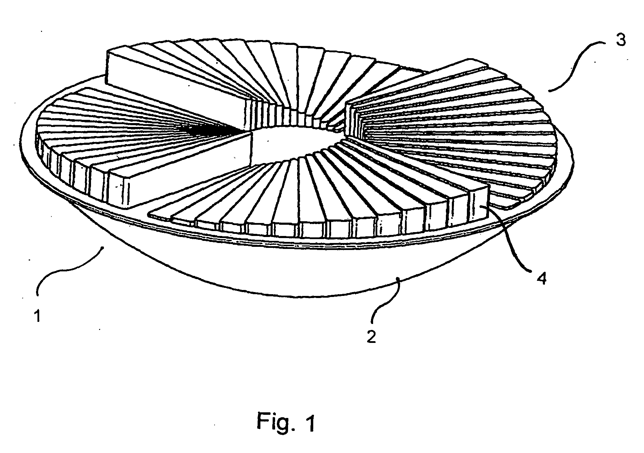

Der untere Teil 2 der Linse 1 in Fig. 1 entspricht einer Standardlinse, d.h. einer Linse für eine optimale Fokussierung. Der obere Teil 3 weist eine nichtrotationssymmetrische Dickenverteilung auf. Diese Form wird auch als Vortex-Linse bezeichnet. Die Linse ist in vier Kreissektoren unterteilt, wobei in dem gezeigten Ausführungsbeispiel jeder Sektor N = 15 radial angeordnete, keilförmige Treppenstufen 4 aufweist, bei denen die Stufenhöhe *h*_{*o*}*,...,h*_{*N*}₋₁*,* innerhalb eines Sektors mit dem Winkel jeweils linear ansteigt. Die nullte Stufe weist eine verschwindende Höhe auf, d.h. *h*₀ = 0.

Die Effekte einer solchen Linse werden im folgenden basierend auf dem Huyghensschen Prinzip beschrieben. Durch die unterschiedliche Höhe der Treppenstufen wird im wesentlichen eine relative zeitliche Verzögerung (Phasenverschiebung) der verschiedenen Elementarwellen erreicht. Die maximale relative zeitliche Verzögerung besteht zwischen den Elementarwellen, welche die höchste Treppenstufe *h*_{*N*}₋₁ und die Treppenstufe mit verschwindender Höhe *h*₀ durchlaufen. Diese Verzögerung wird mit Δ*t*ₘₐₓ bezeichnet.

Bei der gezeigten Anordnung der keilförmigen Treppenstufen ergibt die konstruktive Interferenz pro Sektro einen Fokus. Die Foki sind auf einem Ring angeordnet, dessen Radius R von Δ*t*ₘₐₓ abhängt. Durch geeignete Wahl von Δ*t*ₘₐₓ lässt sich somit die Aufweitung des Fokus beeinflussen.

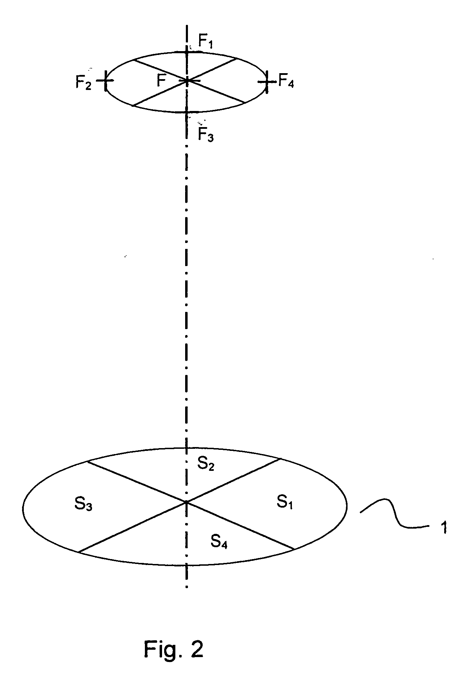

Fig. 2 zeigt die Linse 1 aus Fig. 1 mit vier Sektoren *S*₁,*S*₂,*S*₃ und *S*₄ *,* wobei jeder Sektor N = 15 Treppenstufen umfasst, die in dieser schematischen Darstellung nicht gezeigt sind. Bei einer maximalen Treppenhöhe *h*_{*N*}₋₁ ≠ 0 entsteht pro Sektor ein Fokus *F*₁*,F*₂*,F*₃ und *F*₄ , die alle auf einem Ring mit dem Radius R liegen. Der Radius R ist eine Funktion von *h*_{*N*}₋₁. F bezeichnet den Fokus einer Linse mit maximaler Treppenhöhe *h*_{*N*}₋₁ = 0 .

Zum Beispiel kann die Linse aus Silikongummi mit einer Schallgeschwindigkeit von etwa 950 m/s bestehen. Die Linse befindet sich in Wasser, das eine Schallgeschwindigkeit von 1550 m/s aufweist. Soll beispielsweise die zeitliche Verzögerung zwischen dem Durchgang durch die höchste Stufe gegenüber der Stufe mit verschwindender Höhe 6 µs betragen, was etwa der Länge einer Stoßwelle entspricht, so ergibt sich eine erforderliche maximale Treppenhöhe *h*_{*N*}₋₁ = 15*mm .*

Dem Fachmann ist klar, dass Linsen für einen erfindungsgemäßen Stoßwellengenerator auch in anderer Weise, insbesondere mit anderen Kombinationen von Merkmalen, ausgebildet sein können. Insbesondere kann eine beliebige Anzahl von Sektoren gewählt werden, oder die Phasenverschiebung kann kontinuierlich statt in diskreten Schritten durchgeführt werden, wobei dann N → ∞.

## Patentansprüche

1. Stoßwellengenerator mit einer Linse (1)
**dadurch gekennzeichnet, dass**
die Linse bezüglich der akustischen Achse eine nichtrotationssymmetrische Phasenverschiebungscharakteristik aufweist.

2. Stoßwellengenerator nach Anspruch 1, wobei die nichtrotationssymmetrische Phasenverschiebungscharakteristik nichtkontinuierlich ausgebildet ist.

3. Stoßwellengenerator nach Anspruch 1 oder 2, wobei die Linse wenigstens zwei Sektoren (S₁; S₂; S₃; S₄) mit gleicher Phasenverschiebungscharakteristik aufweist.

4. Stoßwellengenerator nach einem der vorhergehenden Ansprüche, wobei die Linse eine nichtrotationssymmetrische Dickenverteilung zum Ausbilden der nichtrotationssymmetrischen Phasenverschiebungscharakteristik aufweist.

5. Stoßwellengenerator nach Anspruch 4, wobei die Linse eine Mehrzahl von radial ausgerichteten, keilförmigen Elementen (4) zum Ausbilden der nichtrotationssymmetrischen Dickenverteilung aufweist.

6. Stoßwellengenerator nach Anspruch 4 oder 5, wobei die Dicke als Funktion des Winkels über einen vorherbestimmten Winkelbereich linear ansteigt.

7. Stoßwellengenerator nach einem der vorhergehenden Ansprüche, wobei die Linse wenigstens zwei Materialien umfasst.

8. Verwendung einer Linse mit einer nichtrotationssymmetrischen Phasenverschiebungscharakteristik für Stoßwellen.

9. Verwendung nach Anspruch 6, wobei die nichtrotationssymmetrische Phasenverschiebungscharakteristik nichtkontinuierlich ausgebildet ist.

10. Verwendung nach Anspruch 7 oder 8, wobei die Linse eine nichtrotationssymmetrische Dickenverteilung zum Ausbilden der nichtrotationssymmetrischen Phasenverschiebungscharakteristik aufweist.

11. Verwendung nach Anspruch 9, wobei die Linse eine Mehrzahl von radial ausgerichteten, keilförmigen Elementen zum Ausbilden der nichtrotationssymmetrischen Dickenverteilung aufweist.

12. Verwendung nach Anspruch 9 oder 10, wobei die Dicke als Funktion des Winkels über einen vorherbestimmten Winkelbereich linear ansteigt.

13. Verwendung nach einem der Ansprüche 8 - 12, wobei die Linse wenigstens zwei Materialien umfasst.
